# EUROPEAN PATENT APPLICATION

(11) **EP 4 851 267 A1**
(43) Date of publication of application: **09.12.2026**
(21) Application number: 25747039.3
(22) Date of filing: 21.01.2025
(51) Int. Cl.: C11B 1/10, C12N 1/12, C12P 7/6434, C12P 7/6463

(54) **METHOD FOR EXTRACTING USEFUL COMPONENT OF ALGAE**

(30) Priority: 30.01.2024 JP 2024011901
(71) Applicant: Phytolipid Technologies Co., Ltd., Yokohama-shi, Kanagawa, 226-8510 (JP)
(72) Inventor: OHTA, Hiroyuki, Yokohama-shi, Kanagawa 226-8510 (JP); IWAI, Masako, Yokohama-shi, Kanagawa 226-8510 (JP); SASAKI, Yuko, Yokohama-shi, Kanagawa 226-8510 (JP)
(74) Representative: J A Kemp LLP
(86) International application number: PCT/JP2025/001778
(87) International publication number: WO 2025/164444

(57) **Abstract**

Provided is a method for extracting useful components from an alga, the method comprising the following step (1a) and step (2a) or the following step (2b) and step (1b), (1a) a step of extracting a first useful component from a dried alga by using a non-polar solvent, (2a) a step of extracting a second useful component from a residue after the extraction in the step (1a) by using a solvent containing a polar solvent, (2b) a step of extracting a second useful component from a dried alga by using a solvent containing a polar solvent, and (1b) a step of extracting a first useful component from a residue after the extraction in the step (2b) by using a non-polar solvent.

## Description

### Technical Field

The present invention relates to a method for extracting useful components from an alga. This method makes it possible to extract useful components, such as triacylglycerol and carotenoids, and useful components, such as ω-3 fatty acids and oxylipins, separately.

### Background Art

Eustigmatophyceae Nannochloropsis (hereinafter, sometimes referred to as Nannochloropsis), which is a microalga and has high oil productivity, is a marine alga containing various useful components. As findings regarding triacylglycerol (TAG), which has attracted attention as biofuel or food oil and fat, it has been reported that lipids accumulate under nutrient deficient conditions, and among the lipids a storage lipid TAG accumulates, and that the proportion of saturated fatty acids increases (Biotechnol. Bioeng. 102, (2009) 100-112., Frontiers in Microbiology (2015) https://doi.org/10.3389/fmicb.2015.00912). TAG of Nannochloropsis contains a large amount of palmitic acid (C16:0) and is advantageous as a starting material for biodiesel. On the other hand, a large amount of eicosapentaenoic acid (C20:5, EPA), which is an ω-3 fatty acid, is also contained in Nannochloropsis (The Plant Journal (2008) 54, 621-639). Omega-3 fatty acids are mainly contained in large amounts in the membrane lipids of Nannochloropsis, and they are important for various functions in animal bodies and belong to polyunsaturated fatty acid (Frontiers in Microbiology (2015) https://doi.org/10.3389/fmicb.2015.00912). Eicosapentaenoic acid (C20:5, EPA) and DHA, which are ω-3 fatty acids, are contained in marine products such as fatty fish (such as salmon, tuna, and trout) and crustaceans (such as crabs, mussels, and oysters). The ω-3 fatty acids contained in such marine products are not synthesized in the bodies of fish and crustaceans, but ω-3 fatty acids synthesized in the cells of algae are accumulated in the marine products through prey. Marine algae have attracted attention as a new source of ω-3 fatty acids due to fish catches decrease. Furthermore, oxylipins, which are metabolites of EPA, can be produced from Nannochloropsis having EPA in the cell (Phytochemistry 102 (2014) 152-161). It has been reported that these oxylipins widely exhibit antiinflammatory activity (Phytochemistry 102 (2014) 152-161), and a method for extracting a hydroxyeicosapentaenoic acid (HEPE) in krill and shrimp has been reported (Japanese Patent Laid-Open No. 2015-163607). In addition, Nannochloropsis is a natural pigment present in nature and also contains carotenoids known to have antioxidation effects. Carotenoids are contained in large amounts in green and yellow vegetables such as tomatoes and pumpkins, and it has been reported that microalgae also accumulate various carotenoids. For example, carotenoid pigments called diatoxanthin and fucoxanthin are contained in Euglena and brown algae, respectively. Accumulation of carotenoids has also been reported in the genus Nannochloropsis (Algal Research volume 3 (2014) 36-43).

Although Nannochloropsis contains such various useful components, a method for taking out each useful component separately has not been developed yet. In the extraction of lipids from algae, an extraction method using a chloroform-methanol-water mixture is widely used (Non Patent Literatures 1 to 4), but membrane lipids and storage lipids are extracted together in the chloroform-methanol-water extraction, and therefore an operation of further separating useful components is required.

### Citation List

### Non Patent Literature

Non Patent Literature 1
   J Biol Chem 226 (1957) 497-509
Non Patent Literature 2
   Can J Biochem Physiol 37 (1959) 911-917
Non Patent Literature 3
   Eur J Biochem 230 (1995) 987-993
Non Patent Literature 4
   BMC Biotechnology 11:7 (2011) doi: 10.1186/1472-6750-11-7.

### Summary of Invention

### Technical Problem

When useful components contained in an alga can be taken out separately, the recovery work can be made efficient. The present invention has been made in view of such a background, and an object of the present invention is to provide a method for extracting useful components in an alga separately.

### Solution to Problem

The present inventors have conducted intensive studies to solve the above problem to find that useful components, such as TAG and carotenoids, and useful components, such as ω-3 fatty acids and oxylipins, can be extracted separately by using, in two stages, two types of organic solvents, namely, a non-polar solvent and a polar solvent, and have completed the present invention based on this finding.

That is, the present invention provides the following [1] to [7].
[1] A method for extracting useful components from an alga, the method comprising the following step (1a) and step (2a), or the following step (2b) and step (1b):
   (1a) a step of extracting a first useful component from a dried alga by using a non-polar solvent;
   (2a) a step of extracting a second useful component from a residue after the extraction in the step (1a) by using a solvent comprising a polar solvent;
   (2b) a step of extracting a second useful component from a dried alga by using a solvent comprising a polar solvent; and
   (1b) a step of extracting a first useful component from a residue after the extraction in the step (2b) by using a non-polar solvent.
[2] The method according to [1], comprising the step (1a) and the step (2a).
[3] The method according to [1] or [2], wherein the first useful component is triacylglycerol and/or carotenoids, and the second useful component is ω-3 fatty acids and/or oxylipins.
[4] The method according to [1] or [2], wherein the non-polar solvent is hexane, and the solvent containing a polar solvent is ethanol or a mixed solvent of ethanol and hexane.
[5] The method according to [1] or [2], wherein the alga is an alga that comprises triacylglycerol and has a membrane lipid comprising ω-3 fatty acids and oxylipins.
[6] The method according to [1] or [2], wherein the alga is an alga that belongs to the genus Nannochloropsis, the genus Microchloropsis, the genus Monodus, the genus Fistulifera, or the genus Phaeodactylum.
[7] The method according to [1] or [2], wherein the first useful component is triacylglycerol and/or carotenoids, the second useful component is ω-3 fatty acids and/or oxylipins, the non-polar solvent is hexane, the solvent containing a polar solvent is ethanol or a mixed solvent of hexane and ethanol, and the alga is an alga that belongs to the genus Nannochloropsis.

The present specification includes the contents described in the specification and/or drawings of Japanese Patent Application No. 2024-011901, which is the basis of priority of the present application.

### Advantageous Effect of Invention

The present invention provides a novel method for extracting useful components from an alga. This method makes it possible to extract useful components, such as TAG and carotenoids, and useful components, such as ω-3 fatty acids and oxylipins, separately.

### Brief Description of Drawings

[Figure 1] Figure 1 is a diagram showing an outline of a two-stage extraction method.
[Figure 2] Figure 2 shows appearances of extract solutions in each condition.
[Figure 3] Figure 3 is a graph showing composition of fatty acids contained in each extract.
[Figure 4] Figure 4 shows TAG contained in each extract. White spots at a part indicated by each arrow show TAG.
[Figure 5] Figure 5 is a graph showing composition of fatty acids contained in each TAG.
[Figure 6] Figure 6 shows components contained in dried alga.
[Figure 7] Figure 7 shows pigments contained in each extract.
[Figure 8] Figure 8 is a graph showing the amounts of oxylipins contained in second-stage extraction solvents.
[Figure 9] Figure 9 is a diagram showing an outline of operation in Example 2.
[Figure 10] Figure 10 shows appearances of extract solutions in each condition.
[Figure 11] Figure 11 shows TAG contained in each extract. White spots at a part indicated by an arrow show TAG.

### Description of Embodiments

Hereinafter, the present invention will be described in detail.

A method for extracting useful components from an alga of the present invention includes: (1) a step of extracting a first useful component by using a non-polar solvent; and (2) a step of second useful component by using a solvent containing a polar solvent. The order of the step (1) and the step (2) is not particularly limited, and the step (1) may be performed first or the step (2) may be performed first. That is, the method of the present invention may include the following step (1a) and step (2a) or may include the following step (2b) and step (1b).

In the step (1a), the first useful component is extracted from a dried alga by using a non-polar solvent.

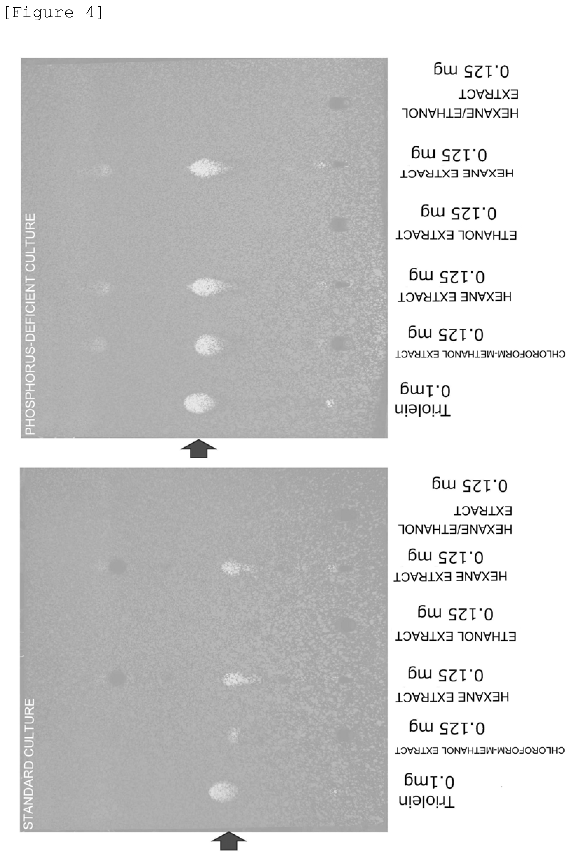

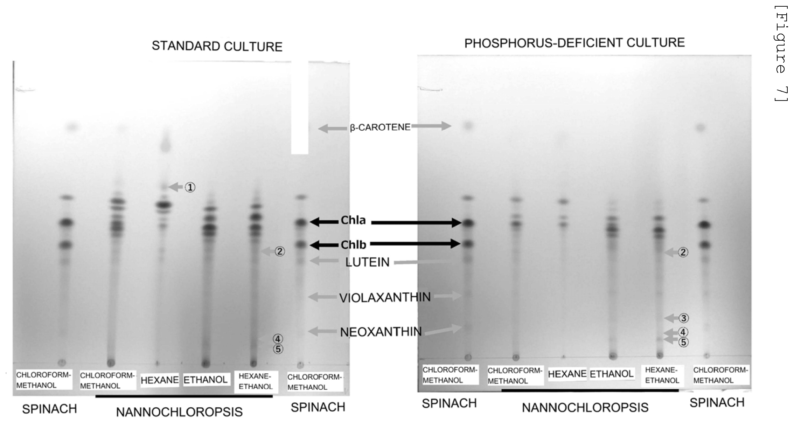

Examples of the non-polar solvent include hexane, cyclohexane, and dichloromethane. Among these, hexane is preferable. This is because by using hexane, almost all the amount of TAG and β-carotene contained in an alga can be recovered (Figure 4 and Figure 7), so that these components can be recovered efficiently. Note that "hexane" in the present invention may be composed of only normal hexane or may contain normal hexane as the main component and a small amount of other materials (for example, methylcyclopentane and methylpentane).

The first useful component is not particularly limited as long as the first useful component can be extracted with a non-polar solvent, but TAG and carotenoids are preferable. Only one of these components may be an extraction target, or both components may be extraction targets. Examples of the carotenoid include β-carotene, α-carotene, γ-carotene, ε-carotene, lutein, zeaxanthin, fucoxanthin, diatoxanthin, and diadinoxanthin.

The alga to be used is not particularly limited as long as the useful components can be extracted therefrom, but an alga belonging to the genus Nannochloropsis, the genus Microchloropsis, the genus Monodus, the genus Fistulifera, or the genus Phaeodactylum is preferable, and an alga belonging to the genus Nannochloropsis is particularly preferable. Examples of the alga belonging to the genus Nannochloropsis include Nannochloropsis oceanica, Nannochloropsis oculata, Nannochloropsis granulata, Nannochloropsis australis, Nannochloropsis limnetica, and Nannochloropsis maritima, examples of the alga belonging to the genus Microchloropsis include Microchloropsis gaditana and Microchloropsis salina, examples of the alga belonging to the genus Monodus include Monodus subterraneus, examples of the alga belonging to the genus Fistulifera include Fistulifera solaris, Fistulifera pelliculosa, and Fistulifera saprophila, examples of the alga belonging to the genus Phaeodactylum include Phaeodactylum tricornutum. In addition, the alga to be used is preferably an alga containing triacylglycerol and having a membrane lipid containing ω-3 fatty acids and oxylipins. Examples of such an alga include an alga belonging to the genus Monodus (for example, Monodus subterraneus), an alga belonging to the genus Fistulifera (for example, Fistulifera solaris, Fistulifera pelliculosa, and Fistulifera saprophila), an alga belonging to the genus Microchloropsis (for example, Microchloropsis gaditana and Microchloropsis salina), and an alga belonging to the genus Nannochloropsis (for example, Nannochloropsis oceanica, Nannochloropsis oculata, Nannochloropsis granulata, Nannochloropsis australis, Nannochloropsis limnetica, and Nannochloropsis maritima).

When an alga belonging to the genus Nannochloropsis is used as the alga, an alga cultured in standard medium may be used, or an alga cultured in phosphorus-deficient medium or nitrogen-deficient medium to accumulate TAG may be used. The accumulation of TAG by using phosphorus-deficient medium can be performed, for example, according to the description in Examples, which will be described later, or the description in International Publication No. WO 2015/137449. The accumulation of TAG by using nitrogen-deficient medium can be performed, for example, according to the description in International Publication No. WO 2015/137449.

The alga can be dried according to a usual method. The useful component can be extracted from the dried alga by a method including, for example, 1) a step of adding the solvent to the dried alga, 2) a step of allowing a certain period of time to elapse, and 3) a step of recovering the supernatant. The amount of the solvent to add is not particularly limited, but 3 to 100 ml of the solvent is preferably added, and 15 to 45 ml of the solvent is more preferably added, based on 100 mg of the dried alga. The time to be allowed to elapse after the addition of the solvent is not particularly limited, but is preferably 0.3 to 24 hours, more preferably 0.5 to 18 hours. The supernatant can be recovered according to a usual method such as centrifugal separation. The above steps 1) to 3) are usually repeated 2 times or more, and a liquid obtained by putting the resulting supernatants together is used as an extract solution of the useful component. The number of repetitions may be 2 or more, but is preferably 3 to 7, more preferably 4 to 6. Components other than the useful component are contained in the above extract solution, and therefore only the useful component may be separated by chromatography or the like.

In the step (2a), the second useful component is extracted from the residue after the extraction in the step (1a) by using a solvent containing a non-polar solvent.

The solvent containing a polar solvent may be any solvent containing a polar solvent and may be a solvent composed of only a polar solvent or a mixed solvent of a polar solvent and a non-polar solvent. Examples of the polar solvent include ethanol, 1-butanol, 2-butanol, 1-propanol, 2-propanol, and acetone. Examples of the non-polar solvent which is mixed with the polar solvent include hexane, cyclohexane, and dichloromethane. Examples of the mixed solvent of a polar solvent and a non-polar solvent include a mixed solvent of ethanol and hexane, a mixed solvent of 1-butanol and hexane, a mixed solvent of 2-butanol and hexane, a mixed solvent of 1-propanol and hexane, a mixed solvent of 2-propanol and hexane, and a mixed solvent of acetone and hexane. The mixing ratio of the polar solvent to the non-polar solvent is not particularly limited but is preferably polar solvent/non-polar solvent = 30/70 to 70/30, more preferably 40/60 to 60/40, in terms of mass ratio. Examples of suitable solvents containing a polar solvent include ethanol, a mixed solvent of ethanol and hexane, a mixed solvent of 1-butanol and hexane, a mixed solvent of 2-butanol and hexane, a mixed solvent of 1-propanol and hexane, a mixed solvent of 2-propanol and hexane, and a mixed solvent of acetone and hexane.

The second useful component is not particularly limited as long as the second useful component can be extracted with a solvent containing a polar solvent, but ω-3 fatty acids and oxylipins are preferable. Only one of these components may be an extraction target, or both components may be extraction targets. Examples of the ω-3 fatty acid include EPA and DHA, and examples of the oxylipin include 15-hydroxyeicosapentaenoic acid, 15-hydroxyeicosatetraenoic acid, 13-hydroxy-9,11-octadecadienoic acid, 13-hydroxy-9,11,15-octadecatrienoic acid, 13-hydroxy-6,9,11-octadecatrienoic acid, 15-hydroxy-8,11,13-eicosatrienoic acid, 8-hydroxyeicosapentaenoic acid, 8-hydroxyeicosatetraenoic acid, and 18-hydroxyeicosapentaenoic acid.

Prior to the extraction in the step (2a), the remaining non-polar solvent is desirably removed from the residue after the extraction in the step (1a). The useful component can be extracted from the residue after the extraction in the step (1a) by a method including, for example, 1) a step of adding the solvent to the residue, 2) a step of allowing a certain period of time to elapse, and 3) a step of recovering the supernatant. The amount of the solvent to add is not particularly limited, but 15 to 100 ml of the solvent is preferably added, and 30 to 60 ml of the solvent is more preferably added, based on 100 mg of the residue. The time to be allowed to elapse after the addition of the solvent is not particularly limited, but is preferably 0.3 to 24 hours, more preferably 0.5 to 18 hours. The supernatant can be recovered according to a usual method such as centrifugal separation. The above steps 1) to 3) are usually repeated 2 times or more, and a liquid obtained by putting the resulting supernatants together is used as an extract solution of the useful component. The number of repetitions may be 2 or more, but is preferably 2 to 6, more preferably 3 to 4. Components other than the useful component are contained in the above extract solution, and therefore only the useful component may be separated by chromatography or the like.

In the step (2b), the second useful component is extracted from the dried alga by using a solvent containing a polar solvent. As for the solvent containing a polar solvent, the same solvent as the one for the step (2a) can be used, and as for the alga, the same alga as the one for the step (1a) can be used. In addition, the second useful component may be the same component as the one in the step (2a).

In the step (1b), the first useful component is extracted from the residue after the extraction in the step (2b) by using a non-polar solvent. As for the non-polar solvent, the same solvent as the one for the step (1a) can be used. The first useful component may the same component as the one in the step (1a).

### Examples

Hereinafter, the present invention will be described in further detail with reference to Examples, but the present invention is not limited to these Examples.

### [Example 1]

### Experimental Material

Eustigmatophyceae Nannochloropsis NIES-2145 (hereinafter referred to as "N. 2145") was used. This algal strain is available from National Institute for Environmental Studies, Independent Administrative Agency (http://www.nies.go.jp).

### Experimental Operation

### 1. Culture Conditions

For the culture of N. 2145, F2N medium was used as a medium for standard liquid culture.

In 100 mL of ion-exchanged water, 440 mg of Na₂EDTA·2H₂O, 316 mg of FeCl₃·6H₂O, 1.2 mg of CoSO₄·7H₂O, 2.1 mg of ZnSO₄·7H₂O, 18 mg of MnCl₂·4H₂O, 0.7 mg of CuSO₄·5H₂O, and 0.7 mg of Na₂MnO₄·2H₂O were dissolved and stored as f/2 metal at 4°C. In 900 mL of ion-exchanged water, 121.14 g of tris(hydroxymethyl)aminomethane was dissolved, the pH of the resulting solution was adjusted to 7.6 with HCL, and then the resulting solution adjusted to 1 L was stored as 1 M Tris-HCL (pH 7.6) at 4°C. In 98.5 mL of artificial seawater, 7.5 mg of NaNO₃, 26.745 mg of NH₄Cl, 3 mg of NaH₂PO₄·2H₂O, 0.25 µg of Vitamin B₁₂, 0.25 µg of Biotin, 50 µg of Thiamine HCl, 0.5 mL of f/2 metal, and 1 mL of 1M Tris-HCl (pH 7.6) were dissolved, and the resulting solution was subjected to filter sterilization and then used as F2N medium. As the artificial seawater, Daigo's Artificial Seawater SP (FUJIFILM) was used. A medium obtained by removing NaH₂PO₄ from F2N medium was used as Phosphorus-deficient medium. Rotary culture was performed at 20 to 30 µmol photons/m²/sec, 23°C, and 120 min⁻¹ using each liquid medium.

### 2. Lipid Extraction

### Cells in Standard Medium

An 850 mL culture solution cultured in standard medium for 20 days was centrifuged at 3,500×g for 5 minutes to pellet the cultured cells. The cultured cells were suspended in 50 mL of ion-exchanged water to remove seawater salt, and then the resulting suspension was centrifuged at 3,500×g for 5 minutes to pellet the cultured cells again. The pellet was suspended in 7 mL of ion-exchanged water, and then the resulting suspension was spread flat and dried at 60°C for 22 hours to obtain 340 mg of a dried alga. Three samples of dried alga (65 mg each) were prepared for the experiments under the following condition 1 to condition 3.

In condition 1, extraction was performed with a chloroform-methanol mixture conventionally used as a control. To 65 mg of the dried alga, 1.5 mL of chloroform and 3 mL of methanol were added, and the resulting mixture was allowed to stand at room temperature for 1 hour while being suspended every 10 minutes. The mixture was centrifuged with a swing rotor at 1,000×g for 5 minutes to recover 4.4 mL of the supernatant as the first extract solution. Further, 1.5 mL of chloroform and 3 mL of methanol were added, and the resulting mixture was allowed to stand at room temperature for 30 minutes while being suspended every 10 minutes. The mixture was centrifuged at 1,000×g for 5 minutes with a swing rotor to recover 5 mL of the supernatant as the second extract solution. Further, 1.5 mL of chloroform and 3 mL of methanol were added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the third extract solution. Further, 3 mL of chloroform and 1.5 mL of methanol were added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fourth extract solution. Finally, 3 mL of chloroform and 1.5 mL of methanol were added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fifth extract solution. The first to fifth extract solutions were combined, dried, dissolved in chloroform:methanol = 2:1 to 25 mg/mL, and then stored at -20°C.

In condition 2, extraction was performed with hexane as the first stage, and after the solvent in the pellet was removed by drying, the second-stage extraction was performed with ethanol. To 65 mg of the dried alga , 4.5 mL of hexane was added, and the resulting mixture was allowed to stand at room temperature for 1 hour while being suspended every 10 minutes. The mixture was centrifuged with a swing rotor at 1,000×g for 5 minutes to recover 4.5 mL of the supernatant as the first extract solution. Further, 4.5 mL of hexane was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the second extract solution. Further, 4.5 mL of hexane was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the third extract solution. Further, 4.5 mL of hexane was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fourth extract solution. Finally, 4.5 mL of hexane was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fifth extract solution. The first to fifth extract solutions were combined, dried, dissolved in hexane to 25 mg/mL, and then stored at -20°C as the first-stage extract. Hexane remaining in the pellet was removed by drying, and then the second-stage extraction was performed using ethanol. To the pellet, 9 mL of ethanol was added, and the resulting mixture was allowed to stand at room temperature for 18 hours. The mixture was centrifuged with a swing rotor at 1,000×g for 5 minutes to recover 9 mL of the supernatant as the first extract solution. Further, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the second extract solution. Further, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the third extract solution. Finally, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the fourth extract solution. The first to fourth extract solutions were combined, dried, dissolved in ethanol to 25 mg/mL, and then stored at -20°C as the second-stage extract.

In condition 3, extraction was performed with hexane as the first stage in the same manner as in condition 2, and after the solvent in the pellet was removed by drying, the second-stage extraction was performed with a hexane-ethanol mixture. The same operation as in condition 2 was performed except that 6 mL of hexane and 3 mL of ethanol were used instead of 9 mL of ethanol.

### Cells in Phosphorus-Deficient Medium

A 3 L culture solution cultured in phosphorus-deficient medium for 7 days was centrifuged at 3,500×g for 5 minutes to pellet the cultured cells. The cultured cells were suspended in 50 mL of ion-exchanged water to remove seawater salt, and then the resulting suspension was centrifuged at 3,500×g for 5 minutes to pellet the cultured cells again. The pellet was suspended in 7 mL of ion-exchanged water, and then the resulting suspension was spread flat and dried at 60°C for 22 hours to obtain 302 mg of a dried alga. Three samples of dried alga (58 mg each) were prepared for the experiments under the following condition 1 to condition 3.

In condition 1, extraction was performed with a chloroform-methanol mixture conventionally used as a control. To 58 mg of the dried alga, 3 mL of chloroform and 6 mL of methanol were added, and the resulting mixture was allowed to stand at room temperature for 1 hour while being suspended every 10 minutes. The mixture was centrifuged with a swing rotor at 1,000×g for 5 minutes to recover 9 mL of the supernatant as the first extract solution. Further, 3 mL of chloroform and 6 mL of methanol were added, and the resulting mixture was allowed to stand at room temperature for 1hour while being suspended every 10 minutes. The mixture was centrifuged at 1,000×g for 5 minutes with a swing rotor to recover 9 mL of the supernatant as the second extract solution. Further, 3 mL of chloroform and 6 mL of methanol were added, and the resulting mixture was allowed to stand at room temperature for 17 hours. The mixture was centrifuged at 1,000×g for 5 minutes with a swing rotor to recover 9 mL of the supernatant as the third extract solution. Further, 6 mL of chloroform and 3 mL of methanol were added, and the resulting mixture was allowed to stand at room temperature for 1 hour while being suspended every 10 minutes. The mixture was centrifuged at 1,000×g for 5 minutes with a swing rotor to recover 9 mL of the supernatant as the fourth extract solution. Further, 6 mL of chloroform and 3 mL of methanol were added, and the resulting mixture was allowed to stand at room temperature for 1 hour while being suspended every 10 minutes. The mixture was centrifuged at 1,000×g for 5 minutes with a swing rotor to recover 9 mL of the supernatant as the fifth extract solution. Further, 6 mL of chloroform and 3 mL of methanol were added, and the resulting mixture was allowed to stand at room temperature for 1 hour while being suspended every 10 minutes. The mixture was centrifuged at 1,000×g for 5 minutes with a swing rotor to recover 9 mL of the supernatant as the sixth extract solution. Finally, 6 mL of chloroform and 3 mL of methanol were added, and the resulting mixture was allowed to stand at room temperature for 1 hour while being suspended every 10 minutes. The mixture was centrifuged at 1,000×g for 5 minutes with a swing rotor to recover 9 mL of the supernatant as the seventh extract solution. In order to shorten the time required for drying, two-layer separation was performed. The lower chloroform layer was recovered as a lipid extract solution. For the two-layer separation, 5.4 mL of 1% (W/V) KCl and 3 mL of chloroform were added to the first to third extract solutions. To the fourth to seventh extract solutions, 5.4 mL of 1% (W/V) KCl and 3 mL of methanol were added. After each extract solution was suspended, the resulting suspension was centrifuged at 1,000×g for 5 minutes with a swing rotor to recover the lower chloroform layer as a lipid extract solution. The first to seventh lipid extract solutions were combined, dried, dissolved in chloroform:methanol = 2:1 to 25 mg/mL, and then stored at -20°C.

In condition 2, extraction was performed with hexane as the first stage, and after the solvent in the pellet was removed by drying, the second-stage extraction was performed with ethanol. To 58 mg of the dried alga , 9 mL of hexane was added, and the resulting mixture was allowed to stand at room temperature for 1 hour while being suspended every 10 minutes. The mixture was centrifuged with a swing rotor at 1,000×g for 5 minutes to recover 9 mL of the supernatant as the first extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the second extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the third extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the fourth extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the fifth extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the sixth extract solution. Finally, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the seventh extract solution. The first to seventh extract solutions were combined, dried, dissolved in hexane to 25 mg/mL, and then stored at -20°C as the first-stage extract. Hexane remaining in the pellet was removed by drying, and then the second-stage extraction was performed using ethanol. To the pellet, 9 mL of ethanol was added, and the resulting mixture was allowed to stand at room temperature for 17 hours. The mixture was centrifuged with a swing rotor at 1,000×g for 5 minutes to recover 9 mL of the supernatant as the first extract solution. Further, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the second extract solution. Further, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the third extract solution. Finally, 9 mL of ethanol was added, and the resulting mixture was allowed to stand at room temperature for 1 hour while being suspended every 10 minutes. The mixture was centrifuged at 1,000×g for 5 minutes with a swing rotor to recover 9 mL of the supernatant as the fourth extract solution. The first to fourth extract solutions were combined, dried, dissolved in ethanol to 25 mg/mL, and then stored at -20°C as the second-stage extract.

In condition 3, extraction was performed with hexane as the first stage in the same manner as in condition 2, and after the solvent in the pellet was removed by drying, the second-stage extraction was performed with a hexane-ethanol mixture. The same operation as in condition 2 was performed except that 6 mL of hexane and 3 mL of ethanol were used instead of 9 mL of ethanol.

### 3. Lipid Analysis

To 5 µl (corresponding to 0.125 mg) of each lipid extract solution, 50 µl of 1 mM heneicosanoic acid and 500 µl of 1.5 M hydrochloric acid/methanol were added, and the resulting mixture was suspended and then allowed to stand still at 85°C for 1 hour for the preparation of fatty acid methyl ester (FAME). To the mixture, 500 µl of hexane was added, and the resulting mixture was suspended and then centrifuged at 1,000×g for 5 minutes with a swing rotor to recover hexane containing the FAMEs in the upper layer. To the lower layer, 500 µl of hexane was added again, and the resulting mixture was suspended and then centrifuged at 1,000×g for 5 minutes with a swing rotor to recover the upper layer. To the lower layer, 500 µl of hexane was added again, and the resulting mixture was suspended and then centrifuged at 1,000×g for 5 minutes with a swing rotor to recover the upper layer. The FAMEs recovered were dried and then dissolved in 100 µl of hexane to make a sample for gas chromatography. Triacylglycerol (TAG) was isolated by thin-layer chromatography (TLC) and then analyzed by gas chromatography. On the TLC plate, 5 µl of the lipid extract solutions (corresponding to 0.125 mg) were spotted and developed for 45 minutes with a mixture of 160 mL of hexane, 40 mL of diethyl ether, and 4 mL of acetic acid. As a control for the mobility of TAG, 0.1 mg of triolein was spotted. TAG was visualized under UV irradiation using 0.001% primulin. The silica on which TAG was present was scraped off, 50 µl of 1 mM heneicosanoic acid and 500 µl of 1.5 M hydrochloric acid/methanol were added thereto, and the resulting mixture was suspended and then allowed to stand still at 85°C for 1 hour for the FAMEs preparation. To the mixture, 500 µl of hexane was added, and the resulting mixture was suspended and then centrifuged at 1,000×g for 5 minutes with a swing rotor to recover hexane containing the FAMEs in the upper layer. To the lower layer, 500 µl of hexane was added again, and the resulting mixture was suspended and then centrifuged at 1,000×g for 5 minutes with a swing rotor to recover the upper layer. To the lower layer, 500 µl of hexane was added again, and the resulting mixture was suspended and then centrifuged at 1,000×g for 5 minutes with a swing rotor to recover the upper layer. The FAMEs recovered were dried and then dissolved in 100 µl of hexane to make a sample for gas chromatography. The gas chromatography was performed using Shimadzu Corporation GC-2030 with HR-SS-10 (inner diameter 0.25 mm, length 25 m)(SHINWA CHEMICAL INDUSTRIES, LTD.) attached thereto.

### 4. Pigment Analysis

Analysis by TLC was performed in order to compare the types of pigments contained in the extracts. On a TLC plate, 10 µl (corresponding to 0.25 mg) of the lipid extract solutions were spotted and developed for 40 minutes with a mixture of 70 mL of petroleum ether and 30 mL of acetone. Pigments obtained by chloroform-methanol extraction from spinach were used as controls.

### 5. Oxylipin Analysis

Analysis was performed using LC-MS in order to examine oxylipins contained in the lipid extract solutions. The lipid extract solution by ethanol for the second stage in condition 2 in which the cells cultured in standard medium and the lipid extract solution by the hexane-ethanol mixture for the second stage in condition 3 were diluted 1,000-fold, and the free 15-HEPE and 15-HETE content in the solutions were measured using Shimadzu Corporation LC-MS 8050. As the column, Phenomenex, Kinetex C8 (inner diameter 2.1 mm, length 150 mm, particle size 2.6 µm) was used.

### Experimental Results

### 1. Lipid Extraction

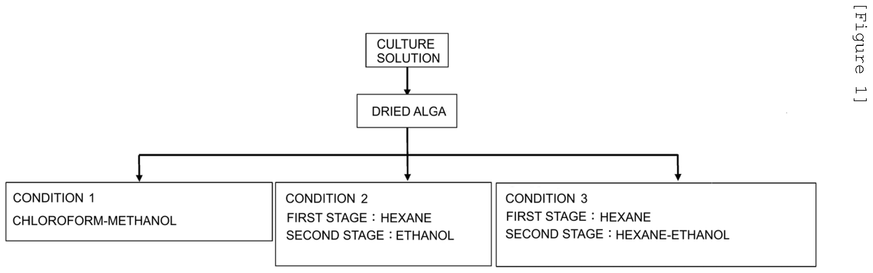

Lipid extraction was performed by the methods of condition 1 to condition 3 in order to compare the conventional extraction with the chloroform-methanol mixture and the two-stage extraction (Figure 1). In condition 1, extraction was performed with a chloroform-methanol mixture conventionally used as a control. In condition 2, extraction was performed with hexane as the first stage, and after the solvent in the pellet was removed by drying, the second-stage extraction was performed with ethanol. In condition 3, extraction was performed with hexane as the first stage, and after the solvent in the pellet was removed by drying, the second-stage extraction was performed with a hexane-ethanol mixture. For lipid extraction, two types of cells were used: the cells cultured in standard medium and the cells cultured in phosphorus-deficient medium, in which TAG more readily accumulates.

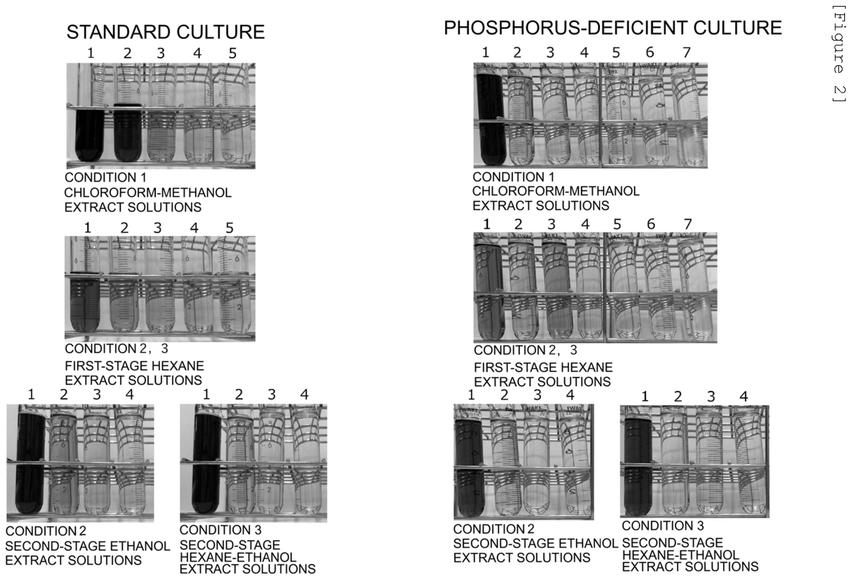

From an 850 mL culture solution cultured with standard medium for 20 days, 340 mg of a dried alga was obtained. Three samples of dried alga (65 mg each) were prepared for the experiments under the following condition 1 to condition 3. Under condition 1, the first extraction yielded a dark green extract solution, the dark green gradually became lighter in the second and third extractions, and the fourth and fifth extract solutions were almost colorless (top left of Figure 2). In the first-stage hexane extraction in conditions 2 and 3, the first extract solution was obtained as a dark green extract solution, and the second to fifth extract solutions did not gradually become lighter in terms of color (middle left of Figure 2). When the second-stage extraction with the hexane-ethanol mixture was performed after the first-stage hexane extraction, there was almost no coloration in the second extraction, but when the extraction was performed with ethanol, the extract solution was colored in green even in the fourth extraction (bottom left of Figure 2). From each weight of lipids recovered, it was found that approximately the same level of lipids as in conventional chloroform-methanol extraction can also be recovered by the two-stage extraction (Table 1).

From a 3 L culture solution cultured with phosphorus-deficient medium for 7 days, 302 mg of a dried alga was obtained. Three samples of dried alga(58 mg each) were prepared for the experiments under the following condition 1 to condition 3. The accumulation amount of TAG was expected to increase in phosphorus-deficient medium, and therefore the amount of organic solvents used for extraction and the number of extractions were increased compared to those for standard medium. Under condition 1, the first extraction yielded a dark green extract solution, the dark green gradually became lighter in the second and third extractions, and the fourth and subsequent extract solutions were colorless (top right of Figure 2). In the first-stage hexane extraction in conditions 2 and 3, the first extract solution, and the third extract solution obtained by extraction for 17 hours were obtained as dark green extract solutions, and the dark green did not gradually become lighter from the fourth extract solution (middle right of Figure 2). When the second-stage extraction with the hexane-ethanol mixture was performed after the first-stage hexane extraction, there was almost no coloration in the second extraction, but when the extraction was performed with ethanol, the extract solution was slightly colored in green even in the fourth extraction (bottom right of Figure 2). From each weight of lipids recovered, it was found that approximately the same level of lipids as in chloroform-methanol extraction can also be recovered from phosphorus-deficient medium-cultured cells, in which the accumulation amount of TAG increases, by the two-stage extraction (Table 1). In addition, it was found that the weight of lipids per weight of dried alga is larger than that from the standard medium-cultured cells. Furthermore, it was found that the amount of lipids recovered in the first stage is larger than that from the standard medium-cultured cells.

**[Table 1]**

| Culture | Condition | Dry weight of alga (mg) | Extraction solvent | Dry weight of extracted lipids (mg) | | Dry weight residue (mg) | Percentage of weight of lipids in weight of dried alga(%) | |
|---|---|---|---|---|---|---|---|---|
| Standard | 1 | 65.07 | Chloroform-methanol | 23.61 | | 48.95 | 36.3 | |
| Standard | 2 | 65.11 | First stage Hexane | First stage | 6.16 | 47.19 | First stage | 9.5 |
| | | | Second stage Ethanol | Second stage | 11.61 | | Second stage | 17.8 |
| Standard | 3 | 65.08 | First stage Hexane | First stage | 5.97 | 46.75 | First stage | 9.2 |
| | | | Second stage Hexane-ethanol | Second stage | 12.49 | | Second stage | 19.2 |
| Phospho rus-deficient | 1 | 58.15 | Chloroform-methanol | 27.61 | | 26.47 | 47.5 | |
| Phospho rus-deficient | 2 | 58.19 | First stage Hexane | First stage | 18.88 | 29.78 | First stage | 32.4 |
| | | | Second stage Ethanol | Second stage | 9.23 | | Second stage | 15.9 |
| Phosphorus -deficient | 3 | 58.23 | First stage Hexane | First stage | 19.19 | 31.30 | First stage | 33.0 |
| | | | Second stage Hexane-ethanol | Second stage | 7.09 | | Second stage | 12.2 |

### 2. Lipid Analysis

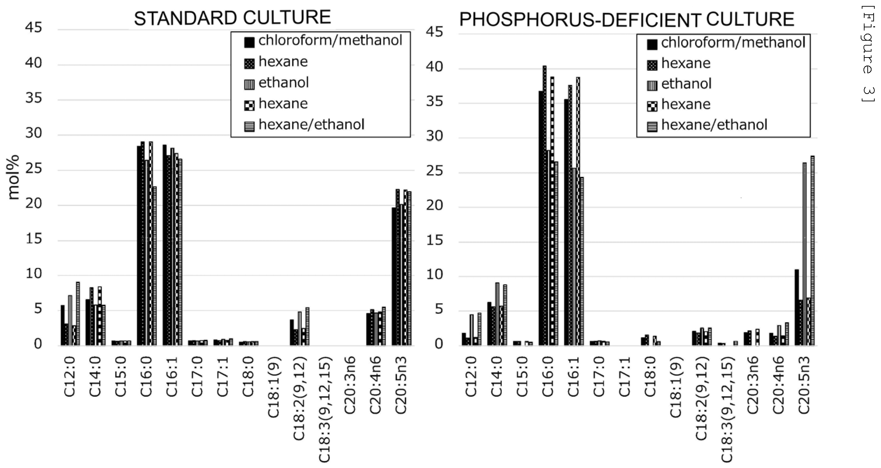

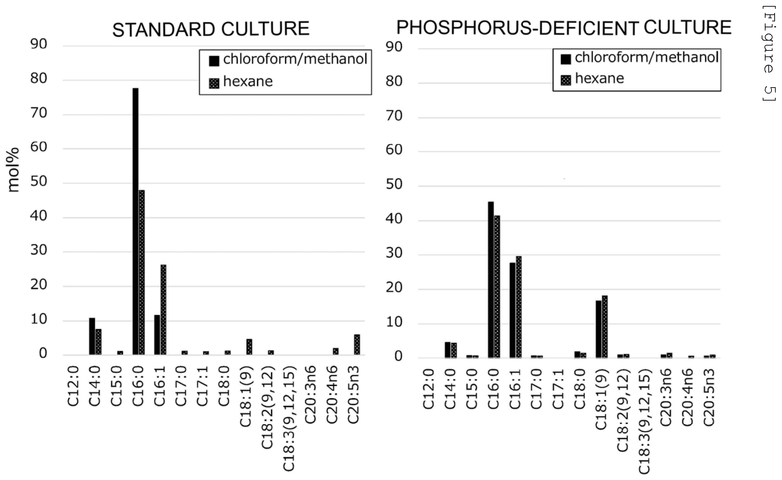

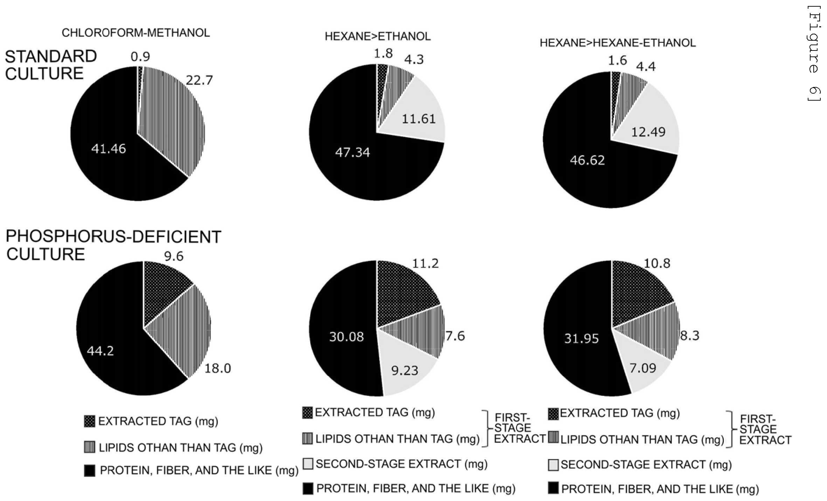

Gas chromatography analysis was performed in order to analyze fatty acids contained in lipids. From the results in Figure 3, regarding the standard culture, the proportion of palmitic acid (C16:0) was smaller in the second-stage ethanol extraction or hexane-ethanol extraction than in chloroform-methanol extraction. The proportion of C16:0 was smaller and the proportion of eicosapentaenoic acid (C20:5, EPA) was larger in hexane-ethanol extraction than in ethanol extraction. Regarding the phosphorus-deficient culture, the proportion of EPA in the first-stage hexane extraction was smaller than in chloroform-methanol extraction, and the proportion of EPA was larger and the proportions of C16:0 and palmitoleic acid (C16:1) were smaller in the second-stage ethanol extraction or hexane-ethanol extraction than in chloroform-methanol extraction. Regarding Nannochloropsis, it is known that the proportions of arachidonic acid (C20:4) and EPA are smaller in TAG than in membrane lipids (Frontiers in Microbiology (2015) https://doi.org/10.3389/fmicb.2015.00912, Plant Physiol. 171,(2016),2469-2482,). It was considered that the reason why the proportion of EPA was smaller in the first stage is because a large amount of TAG is contained in the hexane extract. Thus, TLC analysis and gas chromatography analysis were performed in order to analyze TAG contained in each extract solution. From the results in Figure 4, it was found that the whole amount of TAG can be recovered by the first-stage hexane extraction and TAG is not contained in the second-stage extract solution in both of the standard culture and the phosphorus-deficient culture. In addition, it was revealed that the amount of TAG contained per lipid is larger in hexane extraction than in chloroform-methanol extraction. Fatty acid components contained in each TAG are shown in Figure 5. The amount of TAG in chloroform-methanol extraction for standard culture is small, and therefore EPA could not be detected. Therefore, the proportion of C16:0 was larger than the proportion reported so far, but in hexane extraction, the proportion of EPA was 6%, which is similar to those of fatty acid components contained in TAG after conventional standard culture. Regarding the phosphorus-deficient culture, the proportion of EPA was especially small, as small as 1%. From the above results of the lipid analysis, it was revealed that TAG can be extracted by the first-stage hexane extraction by approximately the same level as or higher than by chloroform-methanol extraction, that TAG is not contained in the second-stage extract, and that by performing ethanol extraction or hexane-ethanol extraction after hexane extraction, lipids can be extracted by approximately the same level as by chloroform-methanol extraction (Figure 6).

### 3. Pigment Analysis

Each extract was spotted on TLC and developed using petroleum ether-acetone. As a result, it was ascertained that β-carotene was contained only in the first-stage hexane extract in the standard culture, and β-carotene was not contained in the second-stage ethanol or hexane-ethanol extract (left of Figure 7). In addition, there were observed 4 spots (indicated by arrows with circled numbers 1, 2, 4 and 5) that seemed like new carotenoids each having mobility which had never been seen for spinach. It was found that in the case of the phosphorus-deficient culture, β-Carotene was contained in the hexane extract, but the amount was smaller than in the case of the standard culture (right of Figure 7). In addition, there was observed a spot that seemed like a new carotenoid (indicated by an arrow with circled number 3) which was not seen in the case of the standard culture. The above results showed that the whole amount of β-carotene can be recovered in the first-stage extract by the two-stage extraction method using a non-polar solvent and a polar solvent of the present invention, that new carotenoids having different characteristics can be recovered separately, and that carotenoids to accumulate can be controlled by culture.

### 4. Oxylipin Analysis

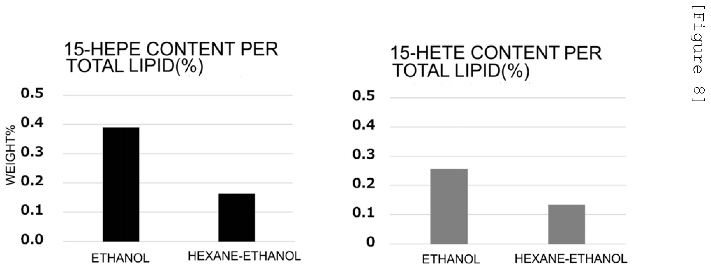

LC-MS was used to examine in which extract oxylipins, which are metabolites of EPA, were contained by the two-stage extraction method. It was found that when ethanol extraction or hexane-ethanol extraction was performed after hexane extraction, free hydroxyeicosapentaenoic acid (15-HEPE) and hydroxyeicosatetraenoic acid (15-HETE) each having affinity for polar solvents were contained in large amounts in the second-stage extract (Figure 8). It was found that oxylipins were recovered in larger amounts by ethanol extraction than by hexane-ethanol extraction.

### 5. Conclusion

The above results showed that the whole amount of TAG and β-carotene can be recovered with the first-stage non-polar solvent by the present invention in which two-stage extraction using a non-polar solvent and a polar solvent is performed after an alga cell is dried. There are two options for the second-stage extraction solvent. When a polar solvent is used, free oxylipins can be efficiently recovered, and when a mixed solvent of a non-polar solvent and a polar solvent is used, the recovery rate of free oxylipins decreases, but the recovery rate of EPA contained in membrane lipids increases. Thus, the present invention is an effective method for recovering useful components separately from an alga according to the purpose.

### [Example 2]

### Experimental Material

The same experimental material as in Example 1 was used.

### Experimental Operation

### Lipid Extraction

A condition in which the order of the extraction steps in condition 2 was reversed was set as condition 4, and an experiment was performed using the cells cultured in standard medium. Specifically, in condition 4, extraction was performed with ethanol as the first stage, and after the solvent in the pellet was removed by drying, the second-stage extraction was performed with hexane. To 65 mg of the dried alga, 4.5 mL of ethanol was added, and the resulting mixture was allowed to stand at room temperature for 1 hour while being suspended every 10 minutes. The mixture was centrifuged with a swing rotor at 1,000×g for 5 minutes to recover 4.5 mL of the supernatant as the first extract solution. Further, 4.5 mL of ethanol was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the second extract solution. Further, 4.5 mL of ethanol was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the third extract solution. Further, 4.5 mL of ethanol was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fourth extract solution. Finally, 4.5 mL of ethanol was added, and the resulting mixture was allowed to stand at room temperature for 30 minutes while being suspended every 10 minutes. The mixture was centrifuged at 1,000×g for 5 minutes with a swing rotor to recover 4.5 mL of the supernatant as the fifth extract solution. The first to fifth extract solutions were combined, dried, dissolved in hexane to 25 mg/mL, and then stored at -20°C as the first-stage extract. Ethanol remaining in the pellet was removed by drying, and then the second-stage extraction was performed using hexane. To the pellet, 9 mL of hexane was added, and the resulting mixture was allowed to stand at room temperature for 18 hours. The mixture was centrifuged with a swing rotor at 1,000×g for 5 minutes to recover 9 mL of the supernatant as the first extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the second extract solution. Further, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the third extract solution. Finally, 9 mL of hexane was added, and the resulting mixture was allowed to stand at room temperature for 1 hour while being suspended every 10 minutes. The mixture was centrifuged at 1,000×g for 5 minutes with a swing rotor to recover 9 mL of the supernatant as the fourth extract solution. The first to fourth extract solutions were combined, dried, dissolved in hexane to 25 mg/mL, and then stored at -20°C as the second-stage extract. For comparison with condition 4, condition 1 and condition 2 were also carried out by the same operation as in Example 1.

### Lipid Analysis

TLC development was performed by the same procedures as in Example 1 in order to examine TAG contained in the extracted lipids.

### Experimental Results

### Lipid Extraction

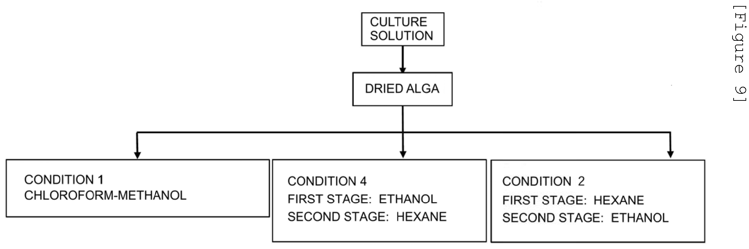

Lipid extractions were performed using the methods of condition 1, condition 2, and condition 4 in order to examine whether extraction is possible when the order of the extraction steps in condition 2 was reversed, which was demonstrated in Example 1 (Figure 9). In condition 1, extraction was performed in the same manner as in Example 1 with a chloroform-methanol mixture conventionally used as a control. In condition 2, extraction was performed with hexane as the first stage, and after the solvent in the pellet was removed by drying, the second-stage extraction was performed with ethanol. In condition 4, extraction was performed with ethanol as the first stage, and after the solvent in the pellet was removed by drying, the second-stage extraction was performed with hexane. For lipid extraction, the cells cultured in standard medium were used.

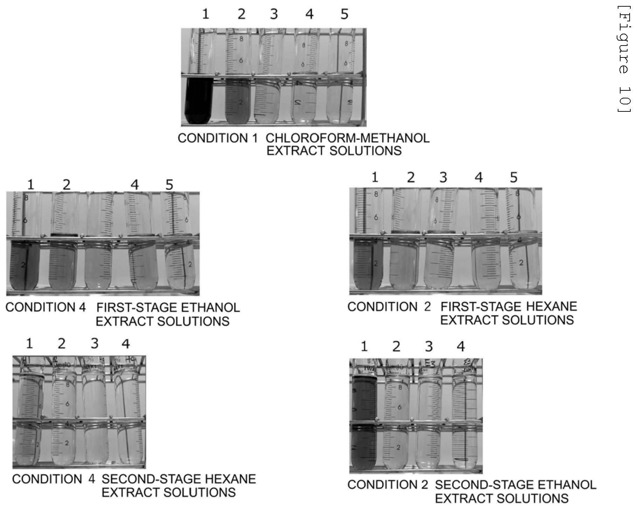

Under condition 1, the first extraction yielded a dark green extract solution, the dark green gradually became lighter in the second and third extractions, and the fourth and fifth extract solutions were almost colorless (top of Figure 10), which was the same as in Example 1.

In the first-stage hexane extraction in conditions 2, the first extract solution was obtained as a dark green extract solution, and the second to fourth extract solutions did not gradually become lighter in terms of color (middle right of Figure 10), which was the same as in Example 1. When the second-stage extraction was performed with ethanol after the first-stage hexane extraction, the extract solution was colored in green even in the fourth extraction (bottom right of Figure 10), which was the same as in Example 1. In the first-stage ethanol extraction in conditions 4, the first extract solution was obtained as a dark green extract solution, and the second to fifth extract solutions did not gradually become lighter in terms of color (middle left of Figure 10). When the second-stage extraction was performed with hexane after the first-stage ethanol extraction, the first extract solution was obtained as a light green extract solution, and there was almost no coloration in the second extraction (bottom left of Figure 10). The second-stage hexane extract solution in condition 4 was lighter in green color than the first-stage hexane extract solution in condition 2. From these results, it was expected that the second-stage hexane extract solution in condition 4 contained smaller amounts of pigment components than the first-stage hexane extract solution in condition 2 and TAG had been able to be recovered efficiently. In addition, from each weight of lipids recovered, it was found that a constant amount of lipids can be recovered even when the order in the two-stage extraction was reversed (Table 2).

**[Table 2]**

| Culture | Condition | Dry weight of alga (mg) | Extraction solvent | Dry weight of extracted lipids (mg) | | Dry weight residue (mg) | Percentage of weight of lipids in weight of dried alga(%) | |
|---|---|---|---|---|---|---|---|---|
| Standard | 1 | 65.0 | Chloroform-methanol | 26.06 | | 30.94 | 40.1 | |
| Standard | 4 | 65.0 | First stage Ethanol | First stage | 7.43 | 48.37 | First stage | 11.4 |
| | | | Second stage Hexane | Second stage | 1.95 | | Second stage | 3.0 |
| Standard | 2 | 65.0 | First stage Hexane | First stage | 4.57 | 46.53 | First stage | 7.0 |
| | | | Second stage Ethanol | Second stage | 6.44 | | Second stage | 9.9 |

### Lipid Analysis

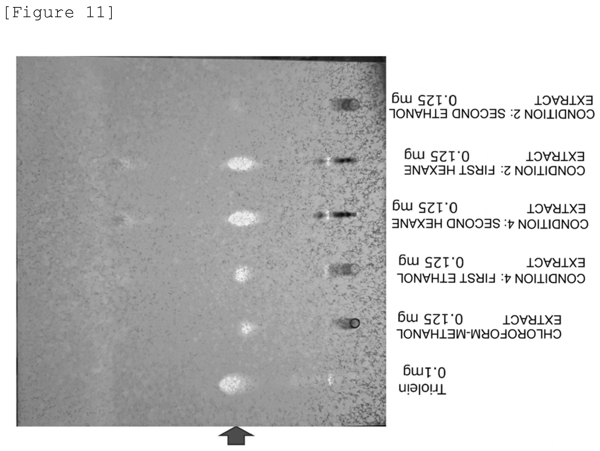

TLC analysis was performed in the same manner as in Example 1 in order to analyze TAG contained in each extract solution. From the results in Figure 11, it was found that under condition 2, the whole amount of TAG can be extracted by the first-stage hexane extraction and TAG was not contained in the second-stage ethanol extract solution, which was the same as in Example 1. Under condition 4 in which the order of the two-stage extraction was reversed, it was revealed that part of TAG is contained in the first-stage ethanol extract solution but most of TAG can be recovered with hexane in the second stage and that the amount of TAG contained per lipid is larger than that by conventional chloroform-methanol extraction.

From the above results of the lipid analysis, it can be said that the first-stage solvent and the second-stage solvent can also be used even if the order was reversed. When the order is reversed, mixing of pigments in the second-stage extraction is reduced and TAG can be recovered more efficiently than by conventional chloroform-methanol extraction.

All publications, patents, and patent applications cited herein are incorporated herein by reference in their entirety.

### Industrial Applicability

TAG that can be extracted by the present invention is used as fuel or food, while ω-3 fatty acids, carotenoids, and oxylipins are utilized as food or medicine. Accordingly, the present invention can be utilized in industrial fields related to fuel, food, medicine, and the like.

## Claims

1. A method for extracting useful components from an alga, the method comprising the following step (1a) and step (2a), or the following step (2b) and step (1b):
(1a) a step of extracting a first useful component from a dried alga by using a non-polar solvent;
(2a) a step of extracting a second useful component from a residue after the extraction in the step (1a) by using a solvent comprising a polar solvent;
(2b) a step of extracting a second useful component from a dried alga by using a solvent comprising a polar solvent; and
(1b) a step of extracting a first useful component from a residue after the extraction in the step (2b) by using a non-polar solvent.

2. The method according to claim 1, comprising the step (1a) and the step (2a).

3. The method according to claim 1 or 2, wherein the first useful component is triacylglycerol and/or carotenoids, and the second useful component is ω-3 fatty acids and/or oxylipins.

4. The method according to claim 1 or 2, wherein the non-polar solvent is hexane, and the solvent containing a polar solvent is ethanol or a mixed solvent of ethanol and hexane.

5. The method according to claim 1 or 2, wherein the alga is an alga that comprises triacylglycerol and has a membrane lipid comprising ω-3 fatty acids and oxylipins.

6. The method according to claim 1 or 2, wherein the alga is an alga that belongs to the genus Nannochloropsis, the genus Microchloropsis, the genus Monodus, the genus Fistulifera, or the genus Phaeodactylum.

7. The method according to claim 1 or 2, wherein the first useful component is triacylglycerol and/or carotenoids, the second useful component is ω-3 fatty acids and/or oxylipins, the non-polar solvent is hexane, the solvent containing a polar solvent is ethanol or a mixed solvent of hexane and ethanol, and the alga is an alga that belongs to the genus Nannochloropsis.
